Europäisches Patentamt

European Patent Office

Office européen des brevets

(11) Publication number: 0 351 924
A2

## (12) EUROPEAN PATENT APPLICATION

(21) Application number: 89201899.5

(22) Date of filing: 18.07.89

(51) Int. Cl.⁴: A01H 5/00 , C12N 15/82 , C12N 15/29 , C12N 1/21

(30) Priority: 20.07.88 GB 8817294
15.11.88 GB 8826663

(43) Date of publication of application:
24.01.90 Bulletin 90/04

(84) Designated Contracting States:
AT BE CH DE ES FR GB IT LI NL SE

(71) Applicant: SHELL INTERNATIONALE
RESEARCH MAATSCHAPPIJ B.V.
Carel van Bylandtlaan 30
NL-2596 HR Den Haag(NL)

(72) Inventor: Hepher, Andrew
10 Front Brents
Faversham Kent(GB)
Inventor: Edwards, Glyn Alyn
University of Durham
South Road Durham(GB)
Inventor: Gatehouse, John Arthur
University of Durham
South Road Durham(GB)

(74) Representative: Bennett, David Arthur Horder
et al
4, York Road
London SE1 7NA(GB)

(54) Improvements relating to transgenic plants.

(57) A transgenic plant such as tobacco or potato comprises a lectin gene expressing a lectin within the plant foreign to the plant as found in nature.

EP 0 351 924 A2

## IMPROVEMENTS RELATING TO TRANSGENIC PLANTS

This invention relates to the expression of lectins in transgenic plants and is particularly, though not exclusively, concerned with the expression of pea lectin in transgenic plants and with novel gene-containing constructs capable of transferring the pea lectin gene in expressible form into plants which do not normally contain the gene.

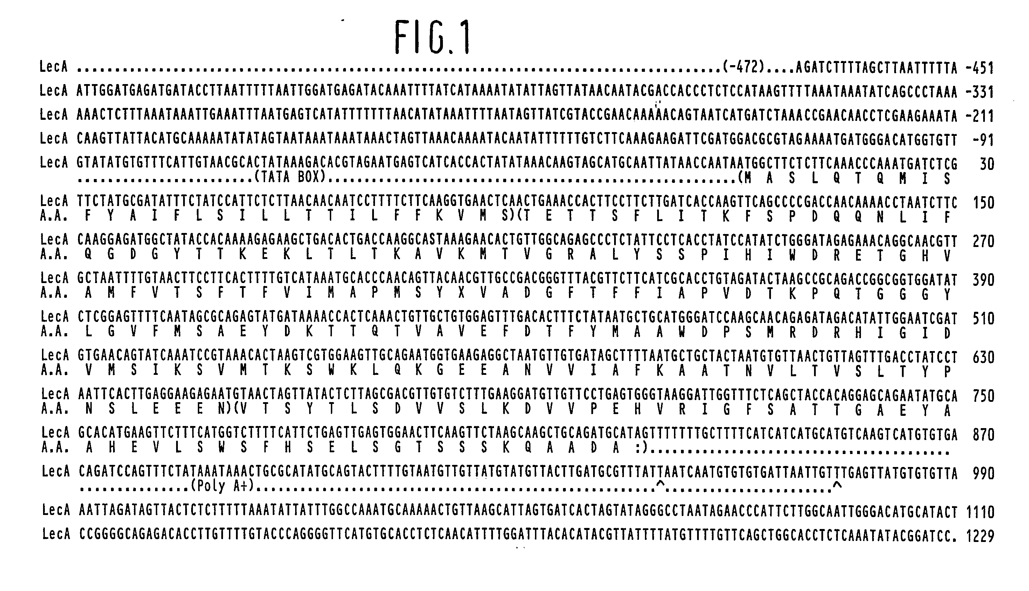

Lectins, which are a group of proteins capable of binding and cross-linking mono- and oligosaccharides, are widely distributed in nature but found most abundantly in the seeds of legumes, although their biological function in such seeds has not been elucidated. An example of such lectins is that derived from pea (Pisum sativum) seeds and the pea lectin gene has been isolated and its base sequence determined - see Gatehouse et al, Nucleic Acids Research, Vol. 15, No. 18, 1987, page 7642. The structure of the genomic clone of the pea lectin gene is given in Fig. 1 which shows the base coding sequence for the lectin, together with, at the 5′ terminal, the naturally occurring promoter sequence and, at the 3′ terminal, the naturally occurring terminator sequence.

It has surprisingly been found that plant lectins such as pea lectin, have pesticidal activity, especially insecticidal activity and that, by suitable genetic manipulation of a lectin genomic clone, it is possible to cause expression of the lectin in a plant which does not naturally contain that lectin at levels suitable for conferring pesticidal activity on the transgenic plant so obtained.

Therefore, in accordance with one form of the invention there is provided a transgenic plant comprising a lectin gene into which has been incorporated a promoter causing the gene to express the lectin within the plant which lectin is foreign to the plant as found in nature. Preferably the plant is a member of the genus Solanaceae; for example it may be a tobacco or potato plant. Preferably the base sequence in the lectin gene coding for the lectin is derived from a member of the genus Leguminoseae, and has a sugar specificity for α-methyl-D-glucose and/or α-methyl-D-mannose monasaccharides (for example the lectin may be Concanavalin A, derived from Canavalia ensiformis, lentil lectin, derived from Lens culinaris or favin, derived from Vicia faba). More preferably the lectin gene carries the coding sequence for pea lectin.

It will be readily apparent to those skilled in the art which promoters will be suitable for causing the gene to express the lectin within the plant, and which genes these promoters may be derived from. It will also be apparent that, depending upon the promoter chosen, the lectin may be expressed either within the whole of the plant or within specific parts of the plant. Preferably the gene is capable of expressing the lectin at least within the leaves of the plant.

Examples of suitable promoters include constitutive promoters (for example promoters derived from Cauliflower Mosaic Virus gene VI, as used by J. Paszkowski et al; EMBO Journal 3, 2717-2722 (1984); Cauliflower Mosaic Virus 35S (CAMV 35S) gene; nopaline synthase gene, as used in AN G. Plant Physiology, 81, 86-91 (1986); and tobacco small-subunit ribulose biphosphate carboxylase (SS Rubisco) gene); leaf specific promoters (for example promoters derived from chlorophyll a/b binding protein gene, as used by J.D.G. Jones et al, Molecular and General Genetics, 212, 536-542 (1988)); wound-inducible promoters (for example promoters derived from potato inhibitor II gene, as used by R.W. Thornburg et al., Proc. Natl. Acad. Sci, 84, 744-748 (1987)); seed-specific promoters (for example promoters derived from pea legumin gene, as used by J.R. Ellis et al; Plant Molecular Biology 10, 203-214 (1988)); tuber-specific promoters (for example derived from patatin gene, as used by D. Twell et al, Plant Molecular Biology, 9, 345-375 (1987)); root-specific promoters (for example promoters derived from glutamine synthase gene, as described in S.V. Tingey et al; EMBO Journal, 6, 1-9 (1987)); or root nodule specific promoters (for example promoters derived from Leghaemoglobin gene, as described in J. Stougaard et al; EMBO Journal, 6, 3565-9 (1987)). Preferably the promoter is derived from cauliflower mosaic virus 35S gene or tobacco small-subunit ribulose biphosphate carboxylase gene.

The invention also includes progeny or propagules derived from such transgenic plants.

The invention also includes a method of making such a transgenic plant comprising infecting the natural plant with a disarmed bacterial strain which has been co-integrated with the lectin gene incorporating the said promoter. Preferably the bacterial strain is a strain of Agrobacterium tumefaciens, suitably the commercially available strain GV3101. When the desired transgenic plant is potato, the co-integrate may, for example, be introduced to potato by incubation with potato mesophyll and subsequent growth of plants from the infected mesophyll. When the desired transgenic plant is tobacco, the co-integrate may, for example, be introduced to tobacco leaves by incubation and subsequent culture of the infected leaf material.

Co-integrates of the bacterial strain and the lectin gene incorporating the said promoter are also included within the scope of the invention.

The construct which is co-integrated with the bacterial strain is suitably obtained by genetic manipulation of a novel chimaeric gene construct comprising a cloning vector into which has been introduced the lectin gene coding sequence having at its 5′ end the desired promoter. Such a novel chimaeric gene construct is also included within the scope of the invention. The cloning vector is selected from suitable commercially available DNA cloning vectors and may be, for example, the standard commercially available DNA cloning vector pUC 18.

The novel chimaeric gene construct can be derived by introduction of the lectin genomic clone initially incorporating its naturally occurring promoter sequence into a standard DNA cloning vector, e.g. pUC 18, removal of the promoter sequence using restriction endonucleases and cloning of the resulting fragment into a plasmid incorporating the desired promoter.

As indicated above the preferred gene coding sequence is that coding for pea lectin. It has been found from the work of Gatehouse et al, (Nucleic Acids Research, Vol. 15, No. 18, 1987, page 7642) that the pea lectin gene has no introns. Furthermore the gene copy number indicated that there was a single copy gene (1 gene/haploid genome). These factors mean that all clones represent active genes which make the pea lectin gene especially suitable for introduction into transgenic plants to give maximum efficiency of gene expression. While pea lectin has been shown to give insecticidal resistance, the invention contemplates the imparting of other pesticidal properties to the transgenic plants dependent on the lectin expressed, for example protective activity against fungi, nematodes and viruses.

The invention will now be further described by way of examples only with reference to the introduction of the pea lectin gene into tobacco and potato plants.

Example 1

Preparation of a Chimaeric Pea Lectin Gene incorporating a promoter derived from Cauliflower Mosaic Virus 35S gene

The starting pea lectin genomic clone as shown in Fig. 1 has a known structure. The clones as described by Gatehouse et al may be employed or clones may be obtained by conventional genetic engineering techniques by building up the structure from the known base sequence or by isolation from pea seeds via a genomic library. The manipulations in the following description were performed in accordance with Maniatis, Fritsch and Sambrook, "Molecular Cloning. A Laboratory Manual", (1982), Cold Spring Harbour Laboratory.

A 1.24 kb DNA fragment from the pea lectin genomic clone in the standard DNA cloning vector pUC 18 was obtained using the restriction endonucleases Sph 1 and Eco R1. The fragment consisted of the pea lectin coding region, 403 base pairs of 3′ sequence (including a polyadenylation site) and 20 base pairs of 5′ untranslated sequence (hereinafter referred to as Lec A). The fragment was cloned into the Sph 1/Eco R 1 sites of pUC 18 to give a plasmid A. Plasmid A comprises Lec A and the 3′ terminator sequence but the native Lec A gene transcriptional start and 5′ controlling sequences have been removed. The ATG translational start is close to the Hind 3 site of the pUC 18 cloning vector.

Plasmid A was digested with Hind 3 and the resulting protruding 5′ termini filled in using the Klenov fragment of E coli DNA polymerase 1. The filled DNA fragment was then digested with enzyme Eco R1 to give a 1.24 kb blunt-ended/Eco R1 fragment. This fragment was cloned into the Sma 1/Eco R1 sites of the commercially available promoter CAMV 35S in the cloning vehicle pUC 18 to give plasmid B. Plasmid B is a chimaeric gene construct in pUC 18 consisting of an 800 base pair fragment containing the CAMV.35S promoter including the CAMV transcriptional start (cap site) together with Lec A. The 5′ untranslated leader sequence between the cap site and the in-frame initial ATG codon of the Lec A coding sequence contains 10 base pairs derived from the CAMV gene, 12 base pairs derived from the pUC 18 cloning region and Klenov filling in step and 20 base pairs derived from the 5′ end of Lec A. This 20 base pair sequence contains an extra ATG codon (out of frame with the Lec A coding sequence) but this did not in fact affect the subsequent expression of the gene.

Example 2

Introduction of Chimaeric Pea Lectin Gene into Tobacco and Potato

A construct C was obtained as follows. A plasmid, which comprises the commercially available plasmid pBR322 together with a kanamycin/gentamycin resistance marker, was ligated with a 1.5kb. Hind III/EcoR1 fragment containing the chimaeric kanamycin resistance construct NOS-NPT II-NOS (supplied by M. Bevan, Institute of Plant Science Research, Cambridge, UK) to give plasmid C1 which contains the sequences necessary for mobilisation to A. tumefaciens. The Bam HI and EcoR1 sites of plasmid C1 were sequentially destroyed by use of restriction enzymes, blunt-ending by Klenov in-filling and re-ligation to give plasmid C2. Plasmid C2 was digested with Hind III, blunt ended and ligated with a 445bp Hae II fragment from the pUC 18 cloning vector containing the lac Z polylinker and to which HhaI linkers had been previously added to give construct C.

Construct C is thus a vector capable of replication in E. coli by virtue of the presence of the commercially available plasmid pBR 322 but not capable of replication in Agrobacterium tumefaciens.

The chimaeric gene construct was cloned from Plasmid B into the Hind 3/Eco R1 sites of construct C to give DNA integration vector D.

A vector E was supplied by Laboratorium voor Genetica, Rijksuniversiteit Gent, B-9000 Gent. Vector E comprised Agrobacterium tumefaciens strain GV 3101 containing the disarmed Ti plasmid pGV 3850 in which the oncogenic functions have been replaced by a sequence from the commercially available plasmid pBR 322 (Zambryski et al, Embo J., 2, pages 2143-2150).

DNA integration vector D and A.tumefaciens-containing vector E were co-integrated and co-integrates selected on YEB-agar containing $100\mu g$ ml$^{-1}$ of both rifampicin and carbenicillin and $5\mu g$ ml$^{-1}$ gentamycin. Southern blots confirmed integration of a single copy of vector D into the T-region of vector E. The co-integrate F so obtained includes the Lec A gene coding sequence, the CAMV promoter and the disarmed A tumefaciens strain and was used to introduce the Lec A gene and consequent expression of pea lectin in tobacco and potato as described below.

Example 3

Production of Transgenic Plants of Tobacco and Potato

A) Potato

Axenic shoot cultures of Solanum tuberosum var DESIREE (obtained from Agricultural Scientific Services, DAFS, East Craigs, Edinburgh) were maintained on MS salts and vitamins, 2% (W/V) sucrose and 0.8% (W/V) purified agar (DIFCO), pH 5.7, by subculturing the shoot apices every 14 days.

Cointegrate F was cultured in dYT medium (16gl$^{-1}$ tryptone, 10gl$^{-1}$ yeast extract, 5gl$^{-1}$ NaCl) containing 5mg l$^{-1}$ gentamycin for 24 h at 27°C. The bacteria were then pelleted, washed three times in 2mM MgSO$_4$ and resuspended in PCM medium (MS salts and vitamins, 2% [W/V] sucrose, 2mg l$^{-1}$ 2,4-dichlorophenoxyacetic acid, 0.5mg l$^{-1}$ zeatin, pH5.7) at a density of approximately 10$^9$ cells ml$^{-1}$.

Excised squares (approximately 7 x 7mm) of potato mesophyll were incubated in the bacterial suspension for 30 minutes with gentle agitation, briefly blotted and transferred, adaxial surface upwards, to PCM medium containing 0.8% purified agar (Difco). After 2 days culture at 25°C (18 hours photoperiod, 120 $\mu$mol m$^{-2}$S$^{-1}$ PAR) explants were washed overnight in PCM containing 100mg l$^{-1}$ Augmentin (Beechams, Worthing, UK), blotted and transferred to solidified PCM containing 100 mg l$^{-1}$ Augmentin and 100 mg l$^{-1}$ kanamycin (acid sulphate). After 4 days culture as before, the leaf explants were transferred to a shoot induction medium (MS Salts + vitamins, 2% [W/V] sucrose, 0.5 mg l$^{-1}$ zeatin, 0.8% purified agar, pH 5.7) containing 100 mg l$^{-1}$ Augmentin and 100 mg l$^{-1}$ kanamycin. After 6 weeks culture transgenic shoots were excised and transferred to the standard shoot culture maintenance medium for rooting. Plantlets were subcultured every 14 days until they had developed to a stage suitable for lectin assay or transplantation to soil (approximately 3 subcultures).

B) Tobacco

Seeds of Nicotiana tabacum cv Petit Havana Str-r were obtained from the Friedrick Miescher Institute, Basel, Switzerland. Plants were grown in Levington Universal compost (Fisons, Ipswich, UK) in a controlled environment of 25°, 70% relative humidity and 18 hour photoperiod (200$\mu$ mol.m$^{-2}$S$^{-1}$ PAR).

Cointegrate F was cultured in dYT medium containing 5mg $1^{-1}$ gentamycin for 24h at 27°C.

The bacteria were then pelleted, washed three times in 2mM MgSO$_4$ and resuspended in TSM medium (MS salts and vitamins, 2% [W/V] sucrose, 1mg $1^{-1}$ 6-benzylaminopurine, 0.1mg $1^{-1}$ naphthaleneacetic acid, pH 5.8) to a density of approximately $10^{10}$ cells ml$^{-1}$.

Expanded leaves of N.tabacum were surface sterilised with 70% (V/V) ethanol (30s) followed by 5% (W/V) calcium hypochlorite (15min). After washing in sterile water, each leaf was cut into squares (approx 10 x 10mm) avoiding major veins and then placed in the bacterial suspension, which was intermittently agitated to wet the cut leaf-edges. After 10-15 min incubation (22-25°C) the leaf pieces were placed (adaxial surface upwards) on TSM medium containing 0.8% (W/V) Difco Bactoagar and cultured at 25°C, 18 hours photoperiod (120 $\mu$ mol m$^{-2}$. S$^{-1}$ PAR). After 2 days, the leaf pieces were transferred to liquid shoot-induction medium containing 1mg ml$^{-1}$ carbencillin (disodium salt) and gently agitated (60rpm) overnight. The leaf pieces were surface-dried on sterile absorbant paper, placed on agar plates of TSM containing kanamycin (acid sulphate) and cultured as before. After approximately 5 weeks, shootlets were transferred to rooting medium comprising half-strength MS salts, 100$\mu$g ml$^{-1}$ carbenicillin, 100$\mu$g ml$^{-1}$ kanamycin and 0.8% (W/V) agar. When the plantlets had developed an extensive root system, they were removed from culture and potted in 1:1 Levington compost: perlite (Silvaperl Products, Harrogate UK) and grown in the controlled environment described above with daily watering.

## Example 4

### Assessment of Level of Pea Lectin in Tobacco and Potato

The quantity of pea lectin expressed in potato and tobacco plants as described in Example 3 above was assessed by enzyme linked immunosorbent assay (ELISA) and radioimmunoassay (RIA). The integrity of the lectin was assessed by Western blotting and the functionality by haemagglutination assay in accordance with the methods described below.

### Methods

#### 1) ELISA ASSAY:

Leaf material was frozen in liquid nitrogen, finely ground, and lyophilised for 12-16 hours. The material was resuspended in extraction buffer (50mM Tris-HCl, pH9.5) and the protein dissolved by shaking at 4°C for 1 hour. The extract was diluted in extraction buffer, and 200$\mu$l of suitable dilutions transferred to the wells of a polystyrene microtitre plate (Nunc Immuno-Fl) along with suitable control dilutions of purified lectin dissolved in extraction buffer. The plates were incubated overnight at 4°C, emptied, and the wells washed 3x with 300$\mu$l phosphate buffered saline (PBS), (8g NaCl, 0.20g KCl, 1.15g Na$_2$HPO$_4$, 0.20g KH$_2$PO$_4$/l), containing 0.1% Tween 20, (this step referred to as "washing" hereafter). 200$\mu$l of PBS containing 1% BSA was added to the wells and the plates incubated for 1 hour, removed and the wells washed. 100$\mu$l primary antibody (rabbit anti-pea lectin) dissolved in PBS containing 1% BSA, 0.1% Tween 20, 10mM MgCl$_2$, 1mM 2-ME, was added to the wells and incubated for 1 hour, removed and the wells washed. 100$\mu$l of $\beta$-galactosidase-conjugated donkey anti-rabbit antibody, dissolved in the same solution as primary antibody, was added to the wells, incubated for 1 hour, removed, and the wells washed. 200$\mu$l of substrate solution (3mM o-nitrophenyl-$\beta$-D-galactopyranoside in PBS containing 3mM MgCl$_2$, 0.1M 2-mercaptoethanol) was added to the wells and the plates incubated at 37°C for up to 3 hours. The O.D.$_{405}$ of the wells was read on a Titertek Multiscan MCC (Flow Laboratories Ltd. UK) plate reader and the concentration of pea lectin in the transformed tissue estimated by comparison to the untransformed controls.

#### 2. RIA ASSAY:

Plant material was harvested, frozen in liquid nitrogen, ground to a fine powder and lyophilised protein was extracted using 50mM Tris-HCl pH 7.5. The protein content of the extract was estimated using a dyebinding assay (Bradford, Analyst-Biochem., 72, pages 248-254), 100$\mu$l of extract at a concentration of 100$\mu$g/ml was filtered through a nitrocellulose filter using a slot blotter (Shliesher and Schnel, Dassel, W. Germany). The filters were then treated as described for the immunological detection of proteins on nitrocellulose by Towbin et al, Proc.Natl. Acad. Sci. USA, 76, pages 4350-4354, using rabbit anti-lectin IgG as primary antibody and I[125]-labelled donkey anti-rabbit IgG as secondary antibody. The filters were autoradiographed and the intensity of the signal determined using a LKB-ultrascan laser densitometer. The signal was compared with standards of pure pea lectin on the filter.

## 3. WESTERN BLOTTING:

Leaf extracts from plants were prepared as for the ELISA assay and extracts containing 200 $\mu$g m$\omega^{-1}$ protein were separated on an 11% non-denaturing polyacrylamide gel. 200 ng purified pea lectin was also run as the positive control. The proteins were transferred to a nitrocellulose filter and detected immunologically.

## 4. HAEMAGGLUTINATION ASSAY:

The haemagglutination activity of plant extracts was assessed according to Sharon and Lis Science, (177), pages 949-959.

Results

Tobacco: Total protein was extracted from fully expanded leaves of 12 individually transformed plants growing in soil, and assessed for pea lectin content using the ELISA assay described above. The results are shown in Table 1 below:

## Table 1

| Transformant | Lectin content (% of total soluble protein) |
|---|---|
| 1 | 0.1 |
| 2 | 0.4 |
| 3 | 0.6 |
| 4 | 0.5 |
| 5 | 0.1 |
| 6 | 0.0 |
| 7 | 0.2 |
| 8 | 0.4 |
| 9 | 0.7 |
| 10 | 0.6 |
| 11 | 0.4 |
| 12 | 0.2 |

Potato: Total protein was extrated from either young unexpanded leaves or fully expanded leaves of 19 individually transformed plants maintained as shoot cultures. Pea lectin content was quantified using the RIA assay described above. The results are shown in Table 2 below:

6

## Table 2

| Transformant | | Lectin content (% of total soluble protein) |
|---|---|---|
| Unexpanded leaves | (1 | 1.8 |
| | (2 | 1.8 |
| | (3 | 0.7 |
| | (4 | 2.0 |
| | (5 | 1.1 |
| Expanded leaves | (6 | 0.0 |
| | (7 | 0.1 |
| | (8 | 0.4 |
| | (9 | 0.1 |
| | (10 | 0.4 |
| | (11 | 0.2 |
| | (12 | 0.1 |
| | (13 | 0.4 |
| | (14 | 0.1 |
| | (15 | 0.0 |
| | (16 | 0.8 |
| | (17 | 0.3 |
| | (18 | 0.0 |
| | (19 | 0.4 |

As can be seen from Tables 1 and 2, expression levels varied between clones and according to the particular tissue sampled. However, using young unexpanded leaves (the primary site of attack for many insects) from the highest expressing lines, expression levels in transformed potato were typically between 1.5 to 2% of total soluble protein. A good correlation was found between the amount of lectin present ( by ELISA or RIA) and activity in the semi-quantitative haemagglutination assay. The fidelity of expression was confirmed by Western blotting, where transgenic pea lectin from both tobacco and potato comigrated with the pea lectin standard, with no significant degradation.

Example 5

Assessment of Insect Toxicity of Pea Lectin

The toxicity of pea lectin was investigated as follows:
Pea lectin was isolated and purified from Pisum sativum L. var FELTHAM FIRST by affinity chromatography using the method described by Pusztai et al, J. Sci. Food Agric., 1977, 28, pages 620-623. Bioassays were carried out with the bean weevil (Callosobruchus maculatus) in accordance with the method described by Gatehouse et al, J. Sci. Food Agric., 1983, 34, pages 345-350.
The results are given in Table 3 below, which gives the percentage adult survival of C. maculatus on an artificial diet containing various levels of purified pea lectin.

Table 3

| Amount lectin in diet (% by weight) | % Adult Survival (after 40 days) |
|---|---|
| 0 (control) | 100.0 |
| 0.5 | 83.4 |
| 1.0 | 79.1 |
| 2.0 | 33.6 |
| 3.5 | 16.5 |
| 5.0 | 5.4 |

It will be seen from the above Table 3 that the pea lectin has an $LD_{50}$ of 1.5% against bean weevil (Callosobrucus masculatus). This correlated with the expression levels of 1.5 to 2% in unexpanded leaves obtained in Example 4 above.

Example 6

Preparation of a Chimaeric Pea Lectin Gene incorporating a promoter derived from tobacco small-subunit ribulose bisphosphate carboxylase (SS Rubisco) gene

Plasmid PBI 130.1 was supplied by M. Bevan, Institute of Plant Science Research, Maris Lane, Trumpington, Cambridge, U.K. Digestion of this plasmid with EcoRI and Hind III yielded a fragment containing the promoter of a tobacco small-subunit ribulose bisphosphate carboxylase (SS-Rubisco) gene and the 3' flanking sequence from the nopaline synthase gene (including the termination/polyadenylation signals). This fragment was cloned into the EcoRI/Hind III sites of pUC18 to give plasmid pDUB131. This was then digested with SmaI and EcoRI, and the large fragment isolated.

Plasmid A was digested with Hind III and the resulting protruding 5' termini filled in using the Klenov fragment of E.coli DNA polymerase I. The blunt-ended DNA fragment was then digested with Eco RI to give a 1.24 kb blunt-ended/EcoRI fragment. This was ligated with the large Sma I/EcoRI fragment from pDUB131, to give plasmid pDUB132. Plasmid pDUB132 is a chimaeric construct in pUC18 consisting of a 1020 base-pair fragment containing the SS-Rubisco promoter (including transcriptional start) together with the Lec A coding sequence and 403 base-pairs of the pea lectin 3'sequence (analogous to plasmid B in Example 1).

Example 7

Expression of chimaeric SS-Rubisco-Lec A construct in transgenic potatoes

Transgenic potato plants were produced according to the methods of Examples 2 and 3 using the pDUB132 plasmid of Example 6. Total protein was extracted from expanded leaves of 45 such individually transformed potato plants maintained as shoot cultures. Pea lectin content was quantified using the RIA assay described in Example 4.

## Table 4

| Transformant | Pea lectin content<br>(% of total soluble protein) |
|:---:|:---:|
| 1 | 0.45 |
| 2 | 0.34 |
| 3 | 0.43 |
| 4 | 0.39 |
| 5 | 0.42 |
| 6 | 0.55 |
| 7 | 0.15 |
| 8 | 1.33 |
| 9 | 0.21 |
| 10 | 0.55 |
| 11 | 0.39 |
| 12 | 0.73 |
| 13 | 0.86 |
| 14 | 0.34 |
| 15 | 0.35 |
| 16 | 0.38 |
| 17 | 0.22 |
| 18 | 0.38 |
| 19 | 0.30 |
| 20 | 0.03 |
| 21 | 0.71 |
| 22 | 0.29 |
| 23 | 0.31 |
| 24 | 0.73 |
| 25 | 0.31 |

## Table 4 (Continued)

| Transformant | Pea lectin content (% of total soluble protein) |
|---|---|
| 26 | 0.65 |
| 27 | 0.85 |
| 28 | 0.62 |
| 29 | 0.45 |
| 30 | 1.01 |
| 31 | 1.23 |
| 32 | 0.55 |
| 33 | 0.54 |
| 34 | 1.48 |
| 35 | 0.29 |
| 36 | 0.30 |
| 37 | 0.49 |
| 38 | 1.01 |
| 39 | 0.36 |
| 40 | 0.34 |
| 41 | 0.76 |
| 42 | 0.65 |
| 43 | 0.12 |
| 44 | 0.08 |
| 45 | 0.51 |

Example 8

Insect bioassay on transformed plants expressing pea lectin

A transformed line of Nicotiana tabacum cv Petit Havana expressing Kanamycin resistance, nopaline synthase and pea lectin (Lec+), and a control transformed line expressing only kanamycin resistance and nopaline synthase (Lec-) were pollinated using a control plant of N. tabacum cv Samsun. Approximately half the progeny from the Lec + maternal parent were shown using RIA to be Lec+, at a level of 0.8 + 0.02 (standard error) percent of total soluble protein. Ten Lec+ progeny and ten progeny from the Lec-maternal parent were used in insect feeding trials to assess resistance to Heliothis virescens.

Eight first instar larvae of H. virescens were added to each plant (approximately 25cm tall) contained within individual sealed planteria. After 7 days the number of surviving insects and their weight was recorded. Additionally, the leaf area consumed was estimated using a 'VIP' image analyser and software supplied by Sightsystems Ltd. (Newbury, U.K.).

The results are summarised in table 5. It is evident that the presence of the pea lectin severely retarded development of the larvae. In addition to being smaller, the larvae on the Lec + plants were either at the first or second instar stage after the 7 day trial, compared with larvae on control Lec-plants that were predominantly third/fourth instar. The reduction in size and maturity of the larvae corresponded with a significantly reduced leaf area consumed.

Table 5

| Effect of expression of pea lectin by transgenic plants on H. virescens larvae. | | | |
|---|---|---|---|
| | surviving insects | per plant (mg) | surviving insect (mg) |
| Lec- | 47 | 45.3 | 9.9 |
| Lec + | 39 | 8.1* | 1.7* |

* significantly different to control lec- plants (p<0.05)

## Claims

1. A transgenic plant comprising a lectin gene into which has been incorporated a promoter causing the gene to express the lectin within the plant which lectin is foreign to the plant as found in nature.

2. A plant according to claim 1 wherein the gene carries the coding sequence for pea lectin.

3. A plant according to claim 1 or 2 which is a tobacco or potato plant.

4. A plant according to claim 1, 2 or 3 wherein the lectin is expressed in the leaves of the plant.

5. A plant according to any one of the preceding claims wherein the promoter is derived from cauliflower mosaic virus 35S gene or tobacco small-subunit ribulose biphosphate carboxylase gene.

6. Progeny or propagules derived from a plant according to any one of claims 1 to 5.

7. A method of making a transgenic plant as claimed in any one of the preceding claims comprising infecting the plant as found in nature with a disarmed bacterial strain which has been co-integrated with the lectin gene incorporating the said promoter.

8. A method according to claim 7 wherein the disarmed bacterial strain is derived from A. tumefaciens.

9. A method according to claim 7 or 8 wherein the plant is potato and the disarmed bacterial strain co-integrated with the lectin gene is introduced to potato by incubation with potato mesophyll and subsequent growth of plants from the infected mesophyll.

10. A method according to claim 7 or 8 wherein the plant is tobacco and the disarmed bacterial strain co-integrated with the lectin gene is introduced to tobacco by incubation with tobacco leaf and subsequent growth of plants from the infected leaf.

11. A co-integrate comprising a disarmed bacterial strain and a lectin gene into which has been incoporated a promoter capable of causing the gene to express a lectin within a plant which lectin is foreign to the plant as found in nature.

12. A co-integrate according to claim 11 wherein the disarmed bacterial strain is derived from A. tumefaciens.

13. A chimaeric gene construct comprising a cloning vector into which has been introduced a lectin gene coding sequence having at its 5' end a promoter capable of causing the gene to express a lectin within a plant, which lectin is foreign to the plant as found in nature.

14. A construct according to claim 13 wherein the cloning vector is DNA cloning vector pUC 18.

15. A co-integrate according to claim 11 or 12 or a construct according to claim 13 or 14 wherein the lectin gene carries the coding sequence for pea lectin.

16. A co-integrate according to claim 11, 12 or 15 or a construct according to claim 13, 14 or 15 wherein the promoter is derived from cauliflower mosaic virus gene or tobacco small-subunit ribulose biphosphate carboxylase gene.

17. A transgenic plant when obtained by the method of any one of claims 7 to 10.

# FIG.1

```
LecA .................................................................................................(-472)....AGATCTTTTAGCTTAATTTTTA -451
LecA ATTGGATGAGATGATACCCTTAATTTTTTAATTGGATGAGATACAAATTTTATCATAAAATATATTAGTTATAACAATACGACCACCCTCTCCATAAGTTTTAAATAAATATCAGCCCTAAA -331
LecA AAACTCTTTAAATAAATTGAAATTTAATGAGTCATATTTTTTTAACATATAAATTTTAATAGTTATCGTACCGAACAAAÄACAGTAATCATGATCTAAACCGAACAACCTCGAAGAAATA -211
LecA CAAGTTATTACATGCAAAAATATATAGTAATAAATAAATAAACTAGTTAAACAAAATACAATATTTTTTGTCTTCAAAGAAGATTCGATGGACGCGTAGAAAATGATGGGACATGGTGTT -91
LecA GTATATGTGTTTCATTGTAACGCACTATAAAGACACGTAGAATGAGTCATCACCACTATATAAACAAGTAGCATGCAATTATAACCAATAATGGCTTCTCTTCAAACCCAAATGATCTCG 30
     ..........................(TATA BOX)...................................................................(M  A  S  L  Q  T  Q  M  I  S
LecA TTCTATGCGATATTTCTATCCATTCTCTTAACAACAATCCTTTTCTTCAAGGTGAACTCAACTGAAACCACTTCCTTCTTGATCACCAAGTTCAGCCCCGACCAACAAAACCTAATCTTC 150
A.A.  F  Y  A  I  F  L  S  I  L  L  T  T  I  L  F  F  K  V  M  S)(T  E  T  T  S  F  L  I  T  K  F  S  P  D  Q  Q  N  L  I  F
LecA CAAGGAGATGGCTATACCACAAAAGAGAAGCTGACACTGACCAAGGCASTAAAGAACACTGTTGGCAGAGCCCTCTATTCCTCACCTATCCATATCTGGGATAGAGAAACAGGCAACGTT 270
A.A.  Q  G  D  G  Y  T  T  K  E  K  L  T  L  T  K  A  V  K  M  T  V  G  R  A  L  Y  S  S  P  I  H  I  W  D  R  E  T  G  H  V
LecA GCTAATTTTGTAACTTCCTTCACTTTTGTCATAAATGCACCCAACAGTTACAACGTTGCCGACGGGTTTACGTTCTTCATCGCACCTGTAGATACTAAGCCGCAGACCGGCGGTGGATAT 390
A.A.  A  M  F  V  T  S  F  T  F  V  I  M  A  P  M  S  Y  X  V  A  D  G  F  T  F  F  I  A  P  V  D  T  K  P  Q  T  G  G  G  Y
LecA CTCGGAGTTTTCAATAGCGCAGAGTATGATAAAACCACTCAAACTGTTGCTGTGGAGTTTGACACTTTCTATAATGCTGCATGGGATCCAAGCAACAGAGATAGACATATTGGAATCGAT 510
A.A.  L  G  V  F  M  S  A  E  Y  D  K  T  T  Q  T  V  A  V  E  F  D  T  F  Y  M  A  A  W  D  P  S  M  R  D  R  H  I  G  I  D
LecA GTGAACAGTATCAAATCCGTAAACACTAAGTCGTGGAAGTTGCAGAATGGTGAAGAGGCTAATGTTGTGATAGCTTTTAATGCTGCTACTAATGTGTTAACTGTTAGTTTGACCTATCCT 630
A.A.  V  M  S  I  K  S  V  M  T  K  S  W  K  L  Q  K  G  E  E  A  N  V  V  I  A  F  K  A  A  T  N  V  L  T  V  S  L  T  Y  P
LecA AATTCACTTGAGGAAGAGAATGTAACTAGTTATACTCTTAGCGACGTTGTGTCTTTGAAGGATGTTGTTCCTGAGTGGGTAAGGATTGGTTTCTCAGCTACCACAGGAGCAGAATATGCA 750
A.A.  N  S  L  E  E  E  N)(V  T  S  Y  T  L  S  D  V  V  S  L  K  D  V  V  P  E  H  V  R  I  G  F  S  A  T  T  G  A  E  Y  A
LecA GCACATGAAGTTCTTTCATGGTCTTTTCATTCTGAGTTGAGTGGAACTTCAAGTTCTAAGCAAGCTGCAGATGCATAGTTTTTTTGCTTTTCATCATCATGCATGTCAAGTCATGTGTGA 870
A.A.  A  H  E  V  L  S  W  S  F  H  S  E  L  S  G  T  S  S  S  K  Q  A  A  D  A  :).................................................
LecA CAGATCCAGTTTCTATAAATAAACTGCGCATATGCAGTACTTTTGTAATGTTGTTATGTATGTTACTTGATGCGTTTATTAATCAATGTGTGTGATTAATTGTTTGAGTTATGTGTGTTA 990
     ................(Poly A+)...................................................^.........................^
LecA AATTAGATAGTTACTCTCTTTTTAAATATTATTTGGCCAAATGCAAAAACTGTTAAGCATTAGTGATCACTAGTATAGGGCCTAATAGAACCCATTCTTGGCAATTGGGACATGCATACT 1110
LecA CCGGGGCAGAGACACCTTGTTTTGTACCCAGGGGTTCATGTGCACCTCTCAACATTTTGGATTTACACATACGTTATTTTATGTTTTGTTCAGCTGGCACCTCTCAAATATACGGATCC. 1229
```